# EUROPEAN PATENT APPLICATION

(11) **EP 4 541 904 A1**
(43) Date of publication of application: **23.04.2025**
(21) Application number: 23823267.2
(22) Date of filing: 16.06.2023
(51) Int. Cl.: C12Q 1/68

(54) **SEQUENCING KIT CONTAINING SULFYDRYL BLOCKING REAGENT AND APPLICATION OF SEQUENCING KIT**

(30) Priority: 16.06.2022 WO PCT/CN2022/099258
(71) Applicant: MGI Tech Co., Ltd., Shenzhen, Guangdong 518083 (CN)
(72) Inventor: JIA, Man, Shenzhen, Guangdong 518083 (CN)
(74) Representative: Calysta NV
(86) International application number: PCT/CN2023/100777
(87) International publication number: WO 2023/241701

(57) **Abstract**

Provided are a sequencing kit containing a sulfydryl blocking reagent and an application of the sequencing kit. Also disclosed is a method for reducing a sequencing background signal. The method comprises the following steps: (1) opening a three-dimensional space structure of the enzyme protein, and exposing an active group; (2) blocking the exposed active group, wherein the blocking is irreversible blocking; (3) releasing a fluorescent substance; and (4) cleaning. For the first time, on the basis of a strategy of opening a disulfide bond of the enzyme protein and then performing irreversible blocking so as to better release the wound dyes substance, a background signal in the sequencing is reduced, and a signal-to-noise ratio and sequencing quality are improved, thereby facilitating efficient high-throughput sequencing.

## Description

The present application claims the right of the priority of Chinese patent application PCT/CN2022/099258 filed on June 16, 2022. The contents of the above Chinese patent application are incorporated herein by reference in their entirety.

### TECHNICAL FIELD

The present disclosure belongs to the field of nucleic acid sequencing, and specifically relates to a kit containing a sulfhydryl blocking reagent, a method for reducing sequencing background signal based on the sulfhydryl blocking reagent, and a use thereof.

### BACKGROUND

DNA sequencing includes first-strand sequencing and second-strand sequencing. After completing the first-strand sequencing, a second strand is generated through multiple displacement amplification (MDA) using the first strand as a template, and then the second strand of DNA is sequenced. During the generation of the second strand, multiplex strand displacement amplification enzyme (phi29 DNA polymerase) is used. Phi29 DNA polymerase is easily and non-specifically adsorbed onto a sequencing chip, making it difficult to remove, leading to residue. The residual phi29 DNA polymerase entangles with dNTPs (chemically modified deoxyribonucleotides) at the beginning stage of second-strand sequencing. Due to the molecular structure of the modified dNTPs containing fluorescent dyes, the entanglement of the non-specifically bound enzyme and dNTPs results in an increase in the background signal during sequencing, causing a decrease in the signal-to-noise ratio, thereby affecting the sequencing quality.

In prior art, the common approach to reduce the adsorption of non-specific fluorescent substances is to elute the chip with an elution solvent. To effectively remove non-specifically bound fluorescent substances, it is necessary to increase the concentration of an eluent and prolong the elution time, yet the effective removal of non-specific adsorption cannot be achieved. Both of these approaches are detrimental to efficient sequencing.

### SUMMARY

In order to solve the technical problem of poor sequencing quality caused by incomplete cleaning of fluorescent dyes in the prior art, the present disclosure provides a sulfhydryl blocking reagent, a method for reducing a sequencing background signal based on the sulfhydryl blocking reagent, and a use thereof.

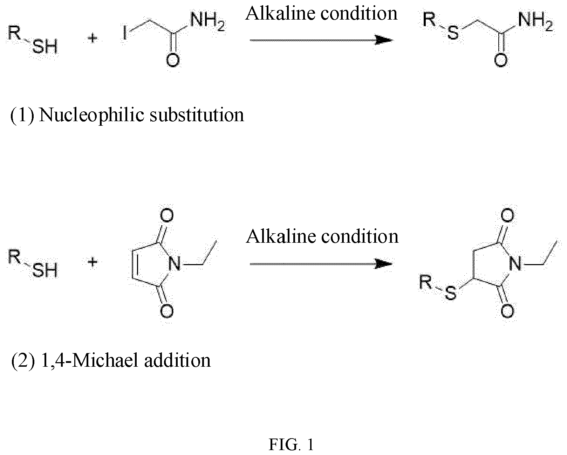

The present disclosure comprises the following steps: first opening a disulfide bond in an enzyme protein structure such that the disulfide bond becomes two exposed sulfhydryl groups, and then blocking the exposed sulfhydryl groups with twoavailable methods. One is halogen substitution reaction, commonly using iodoacetamide, as shown in reaction (1) of FIG. 1. The other is an addition reaction, commonly using N-ethylmaleimide (NEM), as shown in reaction (2) of FIG. 1. Michael addition refers to a conjugated addition reaction between a compound that can provide a nucleophilic carbanion and an electrophilic conjugated system under base catalysis.

The present disclosure uses a disulfide bond reducing agent to open the disulfide bonds in the enzyme protein structure during the sequencing process and uses a blocking reagent to irreversibly block all disulfide bonds, releasing the fluorescent dyes and removing them completely, thereby reducing the background signal and improving the sequencing quality. The sulfhydryl blocking reagent according to the present disclosure includes N-ethylmaleimide and/or iodoacetamide, which are commonly used in protein structures as protein markers and are important tools for protein detection. The present disclosure applies the sulfhydryl blocking reagent in sequencing technology for the first time. As a means to reduce background of second-strand sequencing, the sulfhydryl blocking reagent can effectively block the polymerase during the MDA process, facilitating complete cleaning and preventing its impact on second-strand sequencing, which is conducive to improving the sequencing quality.

In order to solve the above technical problems, the first aspect of the present disclosure provides a sequencing kit, wherein the sequencing kit comprises a sulfhydryl blocking reagent, and the sulfhydryl blocking reagent comprises iodoacetamide and/or *N*-ethylmaleimide.

In certain technical solutions, the sulfhydryl blocking reagent is a 50 mM ammonium bicarbonate solution containing 50 to 100 mM N-ethylmaleimide. Preferably, the sulfhydryl blocking reagent is a 50 mM ammonium bicarbonate solution containing 100 mM N-ethylmaleimide.

In certain technical solutions, the sulfhydryl blocking reagent is a 50 mM ammonium bicarbonate solution containing 50 to 100 mM iodoacetamide. Preferably, the sulfhydryl blocking reagent is a 50 mM ammonium bicarbonate solution containing 100 mM iodoacetamide.

In some technical solutions, the sequencing kit further comprises a disulfide bond reducing agent, wherein the disulfide bond reducing agent is preferably tris(2-carboxyethyl)phosphine (TCEP) or a thiol such as β-mercaptoethanol (β-ME) or dithiothreitol (DTT).

In certain technical solutions, the disulfide bond reducing agent is dithiothreitol with a working solution concentration of 1 to 10 mM.

In some technical solutions, the sequencing kit further comprises one or more of a DNA polymerase, an MDA polymerase, a sequencing slide, and a nucleotide mixture with fluorescent modification and reversible blocking. Preferably, the MDA polymerase is phi29 DNA polymerase.

The second aspect of the present disclosure provides a method for reducing a background signal of nucleic acid sequencing, comprising the following steps:
(1) disrupting the three-dimensional space structure of an enzyme protein during nucleic acid sequencing, and exposing an active group;
(2) blocking the exposed active group with the sulfhydryl blocking reagent in the sequencing kit according to the first aspect of the present disclosure, wherein the blocking is irreversible blocking;
(3) releasing a fluorescent substance;
(4) optionally, cleaning.

In certain technical solutions, in step (1), the three-dimensional space structure is disrupted by breaking a disulfide bond, and the exposed active group is a sulfhydryl group; and/or,
in step (2), the blocking is performed using a separate sulfhydryl blocking reagent or the sulfhydryl blocking reagent in the sequencing kit according to the first aspect; and/or,
in step (3), the fluorescent substance may be a nucleotide individually labeled with a marker, wherein the marker is, for example, Cy5 fluorescence, ROX fluorescence, Cy3 fluorescence, or EF700 fluorescence.

In the present disclosure, "separate" means that the reagent is not derived from the sequencing kit according to the first aspect, but may be an identical reagent prepared separately.

In step (3), the fluorescent substance may be a nucleotide labeled with a combination of markers, allowing different luciferases to be attached to the labeled nucleotide. As used herein, the molecular marker for labeling a nucleotide and the marker that specifically binds thereto may be any pair of molecules that can specifically bind to each other. The specific binding between the paired members enables the linkage of the nucleotide with the luciferase. Exemplary paired members include, but are not limited to: (a) hapten or antigenic compound combined with a corresponding antibody or binding portion or fragment thereof, such as digoxin-digoxin antibody, N3G-N3G antibody, FITC-FITC antibody; (b) nucleic acid aptamer and protein; (c) non-immune binding pair (e.g., biotin-avidin, biotin-streptavidin, biotin-neutravidin); (d) hormone-hormone binding protein; (e) receptor-receptor agonist or antagonist; (f) lectin-carbohydrate; (g) enzyme-enzyme cofactor; (h) enzyme-enzyme inhibitor; and (i) complementary oligonucleotide or polynucleotide pair capable of forming a nucleic acid duplex.

In step (4), the cleaning is performed using an elution reagent, preferably using the elution reagent in the sequencing kit according to the first aspect of the present disclosure.

In certain technical solutions, in step (1), the disulfide bond is reduced using a separate disulfide bond reducing agent or the disulfide bond reducing agent in the sequencing kit according to the first aspect, which may be tris(2-carboxyethyl)phosphine (TCEP), a thiol such as β-mercaptoethanol (β-ME) or dithiothreitol (DTT), so as to break the disulfide bond to open the three-dimensional space. Preferably, the disulfide bond reducing agent has a working solution concentration of 1 to 50 mM, preferably 1 to 10 mM.

In certain technical solutions, in step (2), the sulfhydryl blocking reagent is a 50 mM ammonium bicarbonate solution containing 50 to 100 mM N-ethylmaleimide, either separately or in the sequencing kit according to the first aspect. Preferably, the sulfhydryl blocking reagent is a 50 mM ammonium bicarbonate solution containing 100 mM N-ethylmaleimide.

In certain technical solutions, in step (2), the sulfhydryl blocking reagent is a 50 mM ammonium bicarbonate solution containing 50 to 100 mM iodoacetamide, either separately or in the sequencing kit according to the first aspect. Preferably, the sulfhydryl blocking reagent is a 50 mM ammonium bicarbonate solution containing 100 mM iodoacetamide.

In certain technical solutions, in step (1), an elution of the disulfide bond reducing agent is performed on the sequencing reagent after loading the sequencing reagent containing the disulfide bond reducing agent onto a chip; and/or, in step (2), a secondary elution is performed after adding the sulfhydryl blocking reagent for a reaction.

In certain technical solutions, the elution reagent used for both the elution and secondary elution is an elution reagent in the sequencing reagent tank of an MGISEQ-2000RS high-throughput sequencing kit; and/or, the enzyme protein is an MDA polymerase, such as phi29 DNA polymerase.

The third aspect of the present disclosure provides a method for nucleic acid sequencing, comprising the following steps:
(1) loading a nucleic acid to be tested onto a sequencing slide;
(2) contacting the nucleic acid to be tested with a sequencing reagent, such as dNTP molecules and DNA polymerase, and performing first-strand sequencing;
(3) after completing first-strand sequencing, adding a polymerase for amplification to generate a second strand;
(4) optionally, eluting;
(5) blocking an exposed active group using the sequencing kit according to the first aspect of the present disclosure or the method according to the second aspect of the present disclosure;
(6) optionally, eluting;
(7) performing second-strand sequencing.

Specifically, the nucleic acid sequencing comprises the following steps:
(1) loading DNA nanospheres onto a sequencing slide using an MGISEQ-2000RS high-throughput sequencing kit;
(2) pumping a mixture of dNTP molecules into the sequencing slide, polymerizing the dNTP molecules onto the sequencing strand of DNA using DNA polymerase, and performing first-strand sequencing;
(3) after completing first-strand sequencing, adding MDA polymerase for amplification to generate a second strand, and eluting the excess MDA polymerase and polymerization reagent with an elution reagent;
(4) opening the three-dimensional structure of the residual MDA polymerase on the sequencing chip using the method according to the second aspect of the present disclosure, blocking the exposed group, reacting for a period of, for example, 30 minutes, then eluting the chip with the elution reagent, and performing second-strand sequencing.

Preferably, the nucleic acid sequencing is performed in a gene sequencer MGISEQ-2000RS; the cycle is performed 50 to 400 times, such as 50, 100, 150, 200, or 400 times; and/or, the polynucleotide is DNA or RNA.

The fourth aspect of the present disclosure provides a use of the sequencing kit according to the first aspect in nucleic acid sequencing or in the preparation of a reagent for nucleic acid sequencing.

The positive and progressive effects of the present disclosure are as follows:
The present disclosure uses for the first time a sequencing kit containing a sulfhydryl blocking reagent. On the basis of a strategy of breaking the disulfide bond of the enzyme protein and then performing irreversible blocking so as to better release the entangled dyes substance, the background signal during sequencing is reduced, and the signal-to-noise ratio and sequencing quality are improved. The use of such solutions and reagent components in sequencing protocols and sequencing kits will improve the sequencing quality of the products, thereby increasing their value and market share.

### BRIEF DESCRIPTION OF THE DRAWINGS

FIG. 1 shows the mechanism of irreversible blocking of the sulfhydryl group of DNA polymerase according to the present disclosure.
FIG. 2 shows the results of different blocking reagents on the number of sequencing cycles versus a 0.1% error rate; control group: a 50 mM ammonium bicarbonate solution without any blocking reagent; iodoacetamide: a 50 mM ammonium bicarbonate solution containing 100 mM iodoacetamide; N-ethylmaleimide: a 50 mM ammonium bicarbonate solution containing 50 mM N-ethylmaleimide.
FIG. 3 shows the results of different blocking reagents in reducing the background signal of sequencing; control group: a 50 mM ammonium bicarbonate solution without any blocking reagent; iodoacetamide: a 50 mM ammonium bicarbonate solution containing 100 mM iodoacetamide; N-ethylmaleimide: a 50 mM ammonium bicarbonate solution containing 50 mM N-ethylmaleimide.
FIG. 4 shows the results of different blocking reagents in improving the signal-to-noise ratio of sequencing; control group: a 50 mM ammonium bicarbonate solution without any blocking reagent; iodoacetamide: a 50 mM ammonium bicarbonate solution containing 100 mM iodoacetamide; N-ethylmaleimide: a 50 mM ammonium bicarbonate solution containing 50 mM N-ethylmaleimide.
FIG. 5 shows the Q30 results for different concentrations of N-ethylmaleimide.
FIG. 6 shows the background of A for different concentrations of N-ethylmaleimide.
FIG. 7 shows the background of T for different concentrations of N-ethylmaleimide.
FIG. 8 shows the background of C for different concentrations of N-ethylmaleimide.
FIG. 9 shows the background of G for different concentrations of N-ethylmaleimide.

### DETAILED DESCRIPTION OF THE PREFERRED EMBODIMENT

The present disclosure is further illustrated below by means of examples, but the present disclosure is not thereby limited to the scope of the described examples. The experimental methods for which specific conditions are not indicated in the following examples are selected according to conventional methods and conditions, or according to the product instructions.

The mechanism of irreversible blocking of the sulfhydryl group of DNA polymerase according to the present disclosure is shown in FIG. 1.

### Equipment, reagents, and materials used in examples

### Experimental equipment:

MGISEQ-2000RS sequencer, MGIDL-200H loader, MGISEQ-2000RS sequencing slide. The excitation wavelengths of these instruments are 532 nm and 650 nm, respectively.

Reagents and materials used in experiments (see Table 1):

**Table 1: Reagents and materials used in experiments**

| Sequencing kit | Component | Specification and quantity | Storage temperature | Transport temperature |
|---|---|---|---|---|
| MGISEQ-2000RS sequencing slide Cat. No. 1000008403 | Sequencing slide | 1 EA | -25°C to -15°C | -15°C or less |
| | Low TE Buffer | 300 µL × 1 tube | | |
| | Make DNB Buffer | 100 µL × 1 tube | | |
| | Make DNB Enzyme Mix I | 200 µL × 1 tube | | |
| | Make DNB Enzyme Mix II (LC) | 25 µL × 1 tube | | |
| | Stop DNB Reaction Buffer | 100 µL × 1 tube | | |
| | DNB Load Buffer I | 200 µL × 1 tube | | |
| MGISEQ-2000RS high-throughput sequencing kit (PE150) Cat. No. 1000012537 | DNB Load Buffer II | 200 µL × 1 tube | -25°C to -15°C | -15°C or less |
| | Micro Tube 0.5 mL | 1 tube | | |
| | dNTPs Mix | 1.30 mL × 2 tubes | | |
| | dNTPs Mix II | 1.15 mL × 2 tubes | | |
| | Sequencing Enzyme Mix | 4.80 mL × 1 tube | | |
| | MDA Reagent | 3.50 mL × 1 tube | | |
| | MDA Enzyme Mix | 0.60 mL × 1 tube | | |
| | Sequencing Reagent Cartridge | 1 EA | | |
| | Transparent sealing film | 2 sheets | | |

Ammonium bicarbonate (analytical grade), *N*-ethylmaleimide (NEM, analytical grade), ultrapure water, and *E. coli* single-stranded circular DNA as the template, *i.e.,* standard library reagent V3.0, with primer sequence: CAACTCCTTGGCTCACAGAACATGGCTACGATCCGACTT (SEQ ID NO: 1). DNA polymerase and MDA polymerase were sourced from BGI, and DNA nanospheres were sourced from BGI. The following experiments were all performed using *E. coli* single-stranded circular DNA as the template and using the MGISEQ-2000RS high-throughput sequencing kit (FCL PE150, Cat. No. 1000012555, MGI) to complete the preparation of DNA nanospheres and their loading onto the chip (MGI, Cat. No. 1000008403) for subsequent sequencing. The nucleotide mixture with fluorescent modification and reversible blocking referred to in this example comprises: dATP-1, which refers to an adenine nucleotide modified with both reversible blocking groups and Cy5 fluorescence; dTTP-1, which refers to a thymidine nucleotide modified with both reversible blocking groups and ROX fluorescence; dGTP-1, which refers to a guanine nucleotide modified with both reversible blocking groups and Cy3 fluorescence; and dCTP-1, which refers to a cytosine nucleotide modified with both reversible blocking groups and EF700 fluorescence. The aforementioned dATP-1, dTTP-1, dGTP-1, and dCTP-1 were all sourced from the MGISEQ-2000RS high-throughput sequencing kit. The nucleotide mixture with fluorescent modification and reversible blocking can be varied for different platforms. For example, the nucleotide mixture can be mixed with a nucleotide with only reversible blocking groups or other types of modifications.

### Experimental methods and procedures

(a) Preparation of sulfhydryl blocking reagent:
   A certain amount of ammonium bicarbonate was accurately weighed and dissolved in ultrapure water to prepare a 50 mM ammonium bicarbonate aqueous solution for later use. A certain amount of of *N*-ethylmaleimide and iodoacetamide was then accurately weighed to prepare a 50 mM ammonium bicarbonate solution containing 100 mM iodoacetamide and a 50 mM ammonium bicarbonate solution containing 50 mM *N*-ethylmaleimide.
(b) The method for nucleic acid sequencing follows the instructions, briefly described as follows:
   step 1: loading the DNA nanospheres onto the prepared chip *(i.e.,* sequencing slide);
   step 2: pumping the prepared mixture of dNTP molecules (sourced from the MGISEQ-2000RS high-throughput sequencing kit described above) into the chip, adding the dNTP molecules to the parent strand of DNA using DNA polymerase, and performing first-strand sequencing;
   step 3: after completing first-strand sequencing, proceeding to the MDA process to generate a second strand, and after completing the generation of the second strand, eluting the excess MDA polymerase (sourced from the MGISEQ-2000RS high-throughput sequencing kit described above) with the elution reagent (sourced from the MGISEQ-2000RS high-throughput sequencing kit);
   step 4: pumping in 10 mM DTT reagent to break the disulfide bond of the enzyme protein, and then eluting the DTT reagent; pumping in the sulfhydryl blocking reagent, i.e., ammonium bicarbonate solution containing N-ethylmaleimide, carrying out the reaction for 30 minutes, eluting the solution with the elution reagent, and performing second-strand sequencing.

### Example 1

The MGISEQ-2000RS high-throughput sequencing kit was used, and the #13 well in the kit was added with a 50 mM ammonium bicarbonate solution containing 100 mM iodoacetamide. The nucleotide mixture with fluorescent modification and reversible blocking was sequentially polymerized on the MGISEQ-2000RS sequencing platform, followed by elution of free nucleotides using the elution reagent, allowing for polymerization and compensation during signal collection, at which time the polymerized nucleotide mixture was a nucleotide modified with reversible blocking.

SE50 sequencing was performed on the MGISEQ-2000RS sequencing platform according to the experimental process. In short, the nucleotide mixture with fluorescent modification and reversible blocking was sequentially polymerized on the MGISEQ-2000RS sequencing platform, followed by elution of free nucleotides using the elution reagent, signal collection using the photographic solution, removal of protective groups using the excision reagent, and washing using the elution reagent. The Q30 decline for each cycle and the sequencing error rate curve for each cycle were then calculated to evaluate the sequencing quality.

### Example 2

The MGISEQ-2000RS high-throughput sequencing kit was used, and the #13 well in the kit was added with a 50 mM ammonium bicarbonate solution containing 50 mM N-ethylmaleimide. The nucleotide mixture with fluorescent modification and reversible blocking was sequentially polymerized on the MGISEQ-2000RS sequencing platform, followed by elution of free nucleotides using the elution reagent, allowing for polymerization and compensation during signal collection, at which time the polymerized nucleotide mixture was a nucleotide modified with reversible blocking. SE50 sequencing was performed on the MGISEQ-2000RS sequencing platform according to the same experimental process as that of Example 1. The Q30 decline for each cycle and the sequencing error rate curve for each cycle were then calculated to evaluate the sequencing quality.

### Comparative Example 1

The MGISEQ-2000RS high-throughput sequencing kit was used, and the #13 well in the kit was added with a 50 mM ammonium bicarbonate solution. SE50 sequencing was performed on the MGISEQ-2000RS sequencing platform according to the same experimental process as that of Example 1. The Q30 decline for each cycle and the sequencing error rate curve for each cycle were then calculated to evaluate the sequencing quality.

### Effect Example 1

### Results analysis:

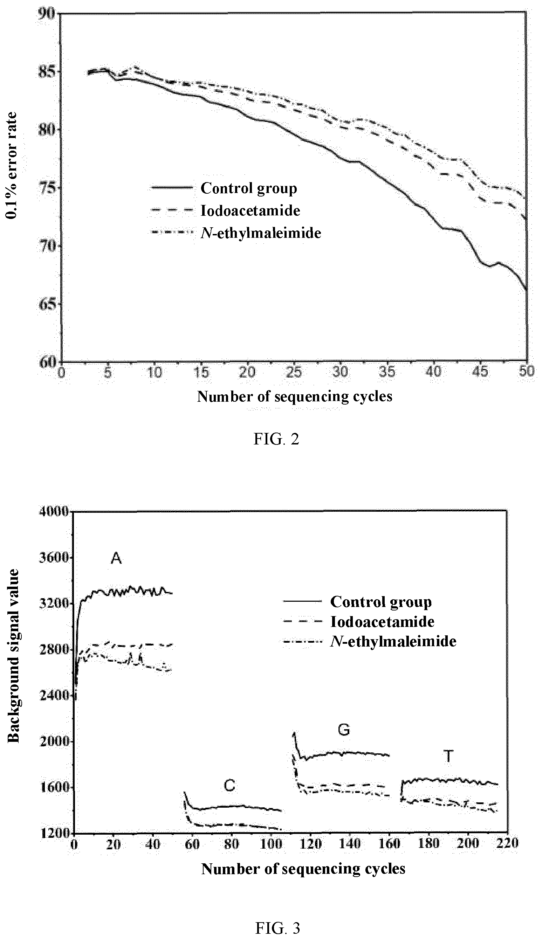

As shown in FIG. 2, the proportion of bases with a 0.1% error rate varies with different blocking reagents during sequencing cycles, and the higher the proportion of bases with a low error rate, the better. Specifically, the 50 mM ammonium bicarbonate solution containing 50 mM N-ethylmaleimide had the highest proportion of bases with a 0.1% error rate; followed by the 50 mM ammonium bicarbonate solution containing 100 mM iodoacetamide; and the 50 mM ammonium bicarbonate solution without any blocking reagent had the lowest proportion of bases with a 0.1% error rate. This indicates that the 50 mM ammonium bicarbonate solution containing 50 mM N-ethylmaleimide has the best effect.

As shown in FIG. 3, during the cycles for A, G, and T, the group using 50 mM ammonium bicarbonate solution containing 50 mM N-ethylmaleimide had the lowest background signal value; followed by the group using 50 mM ammonium bicarbonate solution containing 100 mM iodoacetamide; and the group using 50 mM ammonium bicarbonate solution without any blocking reagent had the highest background signal value. During the cycle for C, the background signal values for the group using 50 mM ammonium bicarbonate solution containing 100 mM iodoacetamide and the group using 50 mM ammonium bicarbonate solution containing 50 mM N-ethylmaleimide were comparable but still lower than that of the group using 50 mM ammonium bicarbonate solution without any blocking reagent.

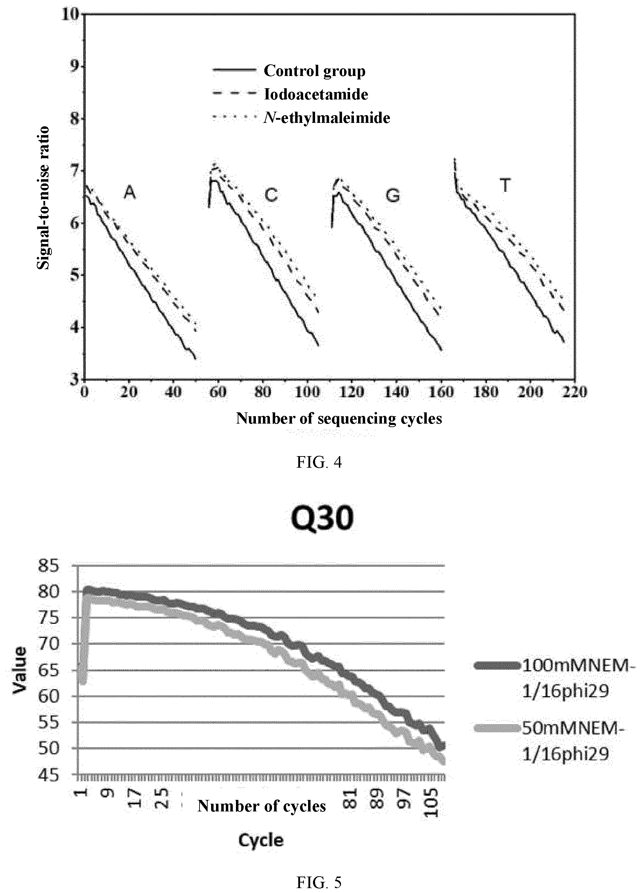

As shown in FIG. 4, during the cycles for A, C, G, and T, the group using 50 mM ammonium bicarbonate solution containing 50 mM N-ethylmaleimide had the highest signal-to-noise ratio; followed by the group using 50 mM ammonium bicarbonate solution containing 100 mM iodoacetamide; and the group using 50 mM ammonium bicarbonate solution without any blocking reagent had the lowest signal-to-noise ratio.

### Effect Example 2

The MGISEQ-2000RS high-throughput sequencing kit was used, and the #13 well in the kit was added with a 50 mM ammonium bicarbonate solution containing 100 mM N-ethylmaleimide and a 50 mM ammonium bicarbonate solution containing 50 mM N-ethylmaleimide, respectively. The nucleotide mixture with fluorescent modification and reversible blocking was sequentially polymerized on the MGISEQ-2000RS sequencing platform, followed by elution of free nucleotides using the elution reagent. SE50 sequencing was performed on the MGISEQ-2000RS sequencing platform according to the same experimental process as that of Example 1. The Q30 decline for each cycle and the sequencing error rate curve for each cycle were then calculated to evaluate the sequencing quality.

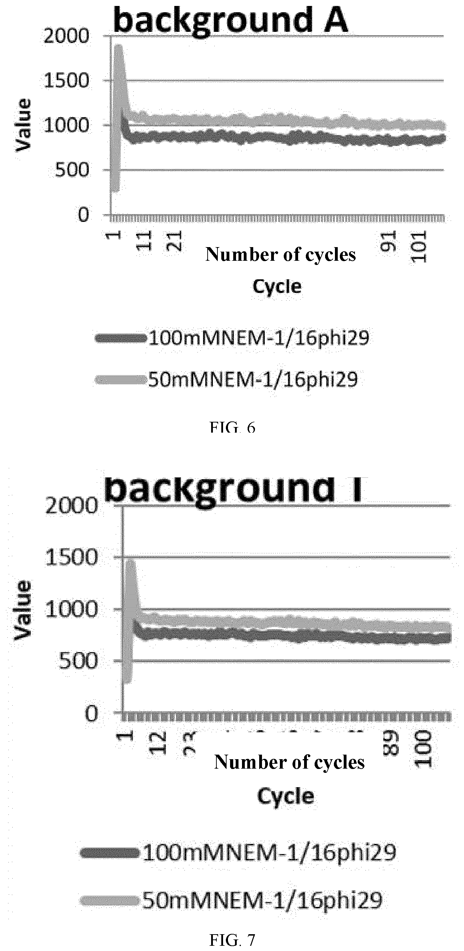

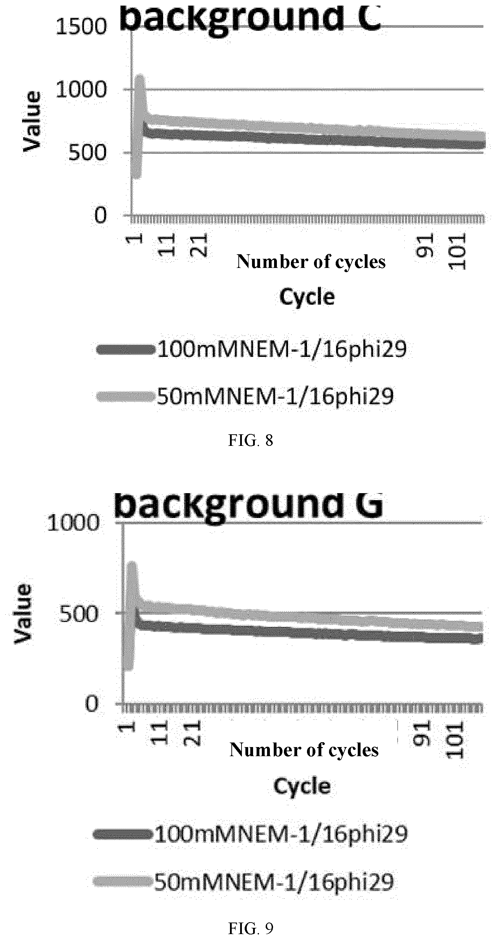

Results: As shown in FIG. 5, the sequencing quality (Q30) was higher when the blocking reagent used was the ammonium bicarbonate solution containing 100 mM *N*-ethylmaleimide than 50 mM *N*-ethylmaleimide. As shown in FIGs 6 to 9, the Q30 (%) for the blocking reagent with a concentration of 100 mM was significantly higher than the sequencing results for the blocking reagent with a component concentration of 50 mM, and the backgrounds for A, G, C, and T were also significantly lower than that of the 50 mM blocking component. Overall, increasing the component concentration of the blocking reagent can lead to better sequencing quality.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A sequencing kit, comprising a sulfhydryl blocking reagent.

2. The sequencing kit according to claim 1, wherein the sulfhydryl blocking reagent comprises iodoacetamide and/or *N*-ethylmaleimide, and/or the sulfhydryl blocking reagent has a concentration of 50 of 100 mM;
preferably, the sulfhydryl blocking reagent is a 50 mM ammonium bicarbonate solution containing 50 to 100 mM N-ethylmaleimide, and/or the sulfhydryl blocking reagent is a 50 mM ammonium bicarbonate solution containing 50 to 100 mM iodoacetamide.

3. The sequencing kit according to claim 1 or 2, wherein the sequencing kit further comprises a disulfide bond reducing agent; the disulfide bond reducing agent is preferably tris(2-carboxyethyl)phosphine or a thiol such as β-mercaptoethanol or dithiothreitol.

4. The sequencing kit according to claim 3, wherein the disulfide bond reducing agent is dithiothreitol with a working solution concentration of 1 to 10 mM.

5. A method for reducing a background signal for nucleic acid sequencing, comprising the following steps:
(1) disrupting a three-dimensional space structure of an enzyme protein during nucleic acid sequencing, and exposing an active group;
(2) blocking the exposed active group with the sulfhydryl blocking reagent as defined in the kit according to any one of claims 1 to 4, wherein the blocking is irreversible blocking;
(3) releasing a fluorescent substance;
(4) optionally, cleaning.

6. The method according to claim 5, wherein in step (1), the three-dimensional space structure is disrupted by breaking a disulfide bond, and the exposed active group is a sulfhydryl group; and/or,
in step (2), the sulfhydryl blocking reagent is a 50 mM ammonium bicarbonate solution containing 50 to 100 mM N-ethylmaleimide, and/or the sulfhydryl blocking reagent is a 50 mM ammonium bicarbonate solution containing 50 to 100 mM iodoacetamide; and/or,
in step (3), the fluorescent substance is a nucleotide individually labeled with a marker, wherein the marker is, for example, Cy5 fluorescence, ROX fluorescence, Cy3 fluorescence, or EF700 fluorescence; and/or the fluorescent substance is a nucleotide labeled with a combination of markers; and/or,
in step (4), the cleaning is performed using an elution reagent.

7. The method according to claim 6, wherein in step (1), the disulfide bond is reduced by a disulfide bond reducing agent such as tris(2-carboxyethyl)phosphine (TCEP) or a thiol such as β-mercaptoethanol (β-ME) or dithiothreitol (DTT), so as to break the disulfide bond to disrupt the three-dimensional space.

8. The method according to claim 3 or 7, wherein in step (1), an elution of the disulfide bond reducing agent is performed on the disulfide bond reducing agent after loading the sequencing reagent containing the disulfide bond reducing agent onto a chip; and/or, in step (2), a secondary elution is performed after adding the sulfhydryl blocking reagent for a reaction.

9. The method according to claim 5, wherein the enzyme protein is an MDA polymerase, such as phi29 DNA polymerase.

10. A method for nucleic acid sequencing, comprising the following steps:
(1) loading a nucleic acid to be tested onto a sequencing slide;
(2) contacting the nucleic acid to be tested with a sequencing reagent, such as dNTP molecules and DNA polymerase, and performing first-strand sequencing;
(3) after completing first-strand sequencing, adding a polymerase for amplification to generate a second strand;
(4) optionally, eluting;
(5) blocking an exposed active group using the sequencing kit according to any one of claims 1 to 4 or the method according to any one of claims 5 to 9;
(6) optionally, eluting;
(7) performing second-strand sequencing.

11. A use of the sequencing kit according to any one of claims 1 to 4 in nucleic acid sequencing or in the preparation of a reagent for nucleic acid sequencing.
